(19) Europäisches Patentamt / European Patent Office / Office européen des brevets

(11) **EP 4 421 663 B1**

(12) **EUROPEAN PATENT SPECIFICATION**

(45) Date of publication and mention
of the grant of the patent:
**03.06.2026   Bulletin 2026/23**

(21) Application number: **22899099.0**

(22) Date of filing: **25.11.2022**

(51) International Patent Classification (IPC):
*G06F 21/32* (2013.01)     *G06V 40/10* (2022.01)
*G06F 21/35* (2013.01)     *A61B 5/00* (2006.01)
*G06F 21/45* (2013.01)     *A61B 5/024* (2006.01)
*A61B 5/117* (2016.01)     *G06F 1/16* (2006.01)
*G06V 40/12* (2022.01)     *G06V 40/16* (2022.01)
*H04L 9/40* (2022.01)

(52) Cooperative Patent Classification (CPC):
**G06F 1/16; A61B 5/024; A61B 5/02416;
A61B 5/02438; A61B 5/117; A61B 5/681;
G06F 1/163; G06F 21/32; G06F 21/35;
G06V 40/1365; G06V 40/15; G06V 40/172;
H04L 63/0861;** G06F 2218/14

(86) International application number:
**PCT/KR2022/018884**

(87) International publication number:
**WO 2023/096417 (01.06.2023 Gazette 2023/22)**

(54) **ELECTRONIC DEVICE AND CONTROL METHOD THEREFOR**

ELEKTRONISCHE VORRICHTUNG UND STEUERUNGSVERFAHREN DAFÜR

DISPOSITIF ÉLECTRONIQUE ET PROCÉDÉ DE COMMANDE ASSOCIÉ

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **26.11.2021   KR 20210166188**

(43) Date of publication of application:
**28.08.2024   Bulletin 2024/35**

(73) Proprietor: **Samsung Electronics Co., Ltd.
Suwon-si, Gyeonggi-do 16677 (KR)**

(72) Inventors:
• **KHAN, Faisal
Dhaka, 1205 (BD)**
• **MOOSA, Ibraheem Muhammad
Dhaka, 1205 (BD)**
• **KHAN, Md. Mahmud Muntakim
Dhaka, 1205 (BD)**

(74) Representative: **Appleyard Lees IP LLP
G Mill
Dean Clough Industrial Park
Halifax HX3 5AH (GB)**

(56) References cited:
JP-A- 2017 070 739     KR-A- 20150 014 788
KR-A- 20150 068 694     KR-A- 20160 075 677
KR-B1- 101 711 835      KR-B1- 101 826 300
US-A1- 2019 332 757     US-A1- 2021 153 757
US-B2- 9 223 956        US-B2- 9 558 336

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

This disclosure relates to an electronic apparatus and a controlling method there of and more particularly, to an electronic apparatus that authenticates a user using a pulse wave signal obtained from the user and a controlling method thereof.

### Background Art

With the development of technology, the functions that can be performed by an electronic apparatus are becoming more diverse, and the time a user spends using an electronic apparatus and the amount of power consumed by the electronic apparatus are also increasing. Accordingly, various technologies for reducing power consumption of an electronic apparatus are continuously being developed, but there is a problem that the technologies developed to date do not sufficiently reflect the user's usage environment or behavior.

In a user authentication system, an electronic apparatus can authenticate a user based on authentication information provided by the user.

In this case, the authentication information may include a password entered by the user or the user's biometric information. Biometric information includes information regarding a fingerprint, iris or face.

However, there is a problem in that an unauthorized user neutralizes the user authentication system of the electronic apparatus 100 in various ways such as imitating the user's biometric information. US 2021/0153757 A1 describes an electronic device including a sensor which measures first biometric information, a communication unit which receives second biometric information measured by at least one wearable device. US 9558336 B2 describes a user-wearable device includes a housing and a band that straps the housing to a portion of a user's body. US 9223956 B2 describes a mobile terminal and a control method thereof.

### Disclosure

### Technical Problem

The present disclosure is to provide an electronic apparatus that authenticates a user by comparing biometric information obtained from a wearable device worn by the user and biometric information obtained by the electronic apparatus and a controlling method thereof.

### Technical Solution

The invention is set out in the appended claims. A controlling method of an electronic apparatus is provided. The method includes, based on a user touch for a first pulse wave sensor included in the electronic apparatus being detected, obtaining a first pulse wave signal through the first pulse wave sensor and receiving a second pulse wave signal detected through a second pulse wave sensor included in a wearable device from the wearable device, removing noise of the first pulse wave signal and the second pulse wave signal using a band pass filter, obtaining a cross-correlation signal indicating a correlation between the denoised first pulse wave signal and the denoised second pulse wave signal, and authenticating the user based on the cross-correlation signal, wherein the receiving comprises: based on the user touch for the first pulse wave sensor (141) included in the electronic apparatus being detected, transmitting time stamp information corresponding to the first pulse wave signal and a signal for requesting the second pulse wave signal to the wearable device; and wherein the second pulse wave signal is obtained based on the time stamp information.

Authenticating the user may include, based on a peak value of the cross-correlation signal being equal to or greater than a first threshold value, authenticating the user.

The method may further include obtaining a deviation score between the denoised first pulse wave signal and the denoised second pulse wave signal by comparing a peak-to-peak interval between the denoised first pulse wave signal and the denoised second pulse wave signal, a pulse interval or a systolic peak, and authenticating the user based on the deviation score.

Authenticating the user may include, based on a deviation score being less than a second threshold value, authenticating the user.

The method may further include identifying whether the user is in a sleeping state, and based on identifying that the user is in a sleeping state, maintaining a locked state of the electronic apparatus regardless of whether the user is authenticated.

The method may further include identifying a heart rate of the user, based on identifying that the heart rate of the user is equal to or less than a third threshold value or equal to or greater than a fourth threshold value, performing a face scan of the user using a camera of the electronic apparatus, identifying whether the user is conscious based on the face scan, and based on identifying that the user is unconscious, maintaining a locked state of the electronic apparatus regardless of whether the user is authenticated.

The method may further include, based on the user authentication being failed while the electronic apparatus is in an unlocked state, switching the electronic apparatus to a locked state.

The method may further include obtaining fingerprint information of the user through a fingerprint sensor included in the electronic apparatus, and the authenticating the user may include, based on the fingerprint information matching fingerprint information stored in the electronic apparatus and the peak value being equal to or greater than a first threshold value, authenticating the user.

The method may further include identifying a user by comparing the first pulse wave signal or the second pulse wave signal with a third pulse wave signal stored in the electronic apparatus.

An electronic apparatus according to an embodiment includes a first pulse wave sensor, a communication interface, a memory configured to store at least one instruction, and a processor connected to the memory and configured to control the electronic apparatus, and the processor may be configured to, by executing the at least one instruction, cause the electronic apparatus to: based on a user touch for the first pulse wave sensor being detected, obtain a first pulse wave signal through the first pulse wave sensor and receive a second pulse wave signal detected through a second pulse wave sensor included in a wearable device from the wearable device, remove noise of the first pulse wave signal and the second pulse wave signal using a band pass filter, obtain a cross-correlation signal indicating a correlation between the denoised first pulse wave signal and the denoised second pulse wave signal, and authenticate the user based on the cross-correlation signal, wherein the processor is configured to cause the apparatus to, based on the user touch for the first pulse wave sensor (141) included in the electronic apparatus being detected, transmit time stamp information corresponding to the first pulse wave signal and a signal for requesting the second pulse wave signal to the wearable device, and wherein the second pulse wave signal is obtained based on the time stamp information.

The processor may be configured to cause the electronic apparatus to, based on a peak value of the cross-correlation signal being equal to or greater than a first threshold value, authenticate the user.

The processor may be configured to cause the electronic apparatus to obtain a deviation score between the denoised first pulse wave signal and the denoised second pulse wave signal by comparing a peak-to-peak interval between the first pulse wave signal and the second pulse wave signal, a pulse interval or a systolic peak, and authenticate the user based on the deviation score.

The processor may be configured to cause the electronic apparatus to, based on the deviation score being less than a second threshold value, authenticate the user.

The processor may be configured to cause the electronic apparatus to identify whether the user is in a sleeping state, and based on identifying that the user is in a sleeping state, maintain a locked state of the electronic apparatus regardless of whether the user is authenticated.

The processor may be configured to cause the electronic apparatus to identify a heart rate of the user, based on identifying that the heart rate of the user is equal to or less than a third threshold value or equal to or greater than a fourth threshold value, perform a face scan of the user using a camera of the electronic apparatus, identify whether the user is conscious based on the face scan, and based on identifying that the user is unconscious, maintain a locked state of the electronic apparatus regardless of whether the user is authenticated.

The processor may be configured to cause the electronic apparatus to, based on the user authentication being failed while the electronic apparatus is in an unlocked state, switch the electronic apparatus to a locked state.

The processor may be configured to cause the electronic apparatus to obtain fingerprint information of the user through a fingerprint sensor included in the electronic apparatus, and based on the fingerprint information matching fingerprint information stored in the electronic apparatus and the peak value being equal to or greater than a first threshold value, authenticate the user.

In a computer readable recording medium including a program for executing a controlling method of an electronic apparatus according to an embodiment, the controlling method of the electronic apparatus includes based on a user touch for a first pulse wave sensor included in the electronic apparatus being detected, obtaining a first pulse wave signal through the first pulse wave sensor and receiving a second pulse wave signal detected through a second pulse wave sensor included in a wearable device from the wearable device, removing noise of the first pulse wave signal and the second pulse wave signal using a band pass filter, obtaining a cross-correlation signal indicating correlation between the first pulse wave signal and the second pulse wave signal, and authenticating the user based on the cross-correlation signal.

**Advantageous Effect**

Through the above-described embodiments, by comparing a pulse wave signal obtained from a wearable device and a pulse wave signal obtained from an electronic apparatus, there is an effect of preventing the problem of a user authentication system being neutralized by an authenticated user operating the electronic apparatus and improving the security of the user authentication system.

**Description of Drawings**

FIGS. 1A and 1B are views provided to describe a user authentication system according to an embodiment;

FIG. 2 is a block diagram provided to describe configuration of an electronic apparatus according to an embodiment;

FIG. 3 is a view provided to describe a method for obtaining a pulse wave signal and fingerprint information according to an embodiment;

FIG. 4 is a view provided to describe a method of performing user authentication using a cross-correlation signal of a first pulse wave signal and a second pulse wave signal according to an embodiment;

FIGS. 5A and 5B are views provided to describe a method of performing user authentication using a cross-correlation signal of a first pulse wave signal and a second pulse wave signal according to an embodiment;

FIG. 6 is a view provided to describe a method of performing user authentication using a deviation score between a first pulse wave signal and a second pulse wave signal according to an embodiment;

FIG. 7 is a view provided to describe a method of performing user authentication using a deviation score between a first pulse wave signal and a second pulse wave signal according to an embodiment;

FIG. 8 is a view provided to describe a controlling method of an electronic apparatus according to whether a user is in a sleeping stat according to an embodiment;

FIG. 9 is a view provided to describe a controlling method of an electronic apparatus according to whether a user is conscious according to an embodiment; and

FIG. 10 is a view provided to describe a method of performing user authentication of an electronic apparatus according to an embodiment.

**Mode for Invention**

The disclosure may be variously modified and have various embodiments. Therefore, specific embodiments are shown in the accompanying drawings and described in detail in the specification. However, it is to be understood that the scope of the disclosure is not limited to the specific embodiments, and includes various modifications, equivalents, or alternatives according to the embodiments of the disclosure. Throughout the accompanying drawings, similar components are denoted by similar reference numerals.

In describing the disclosure, the description omits a detailed description of a case where the detailed description for the known functions or configurations related to the disclosure is determined to unnecessarily obscure the gist of the disclosure.

In addition, the following embodiments may be modified in various different forms, and the scope of the disclosure are not limited to the following embodiments. Rather, these embodiments are provided to make the disclosure thorough and complete.

Terms used in the disclosure are used only to describe the specific embodiments rather than limiting the scope of the disclosure. A term of a singular number may include its plural number unless explicitly indicated otherwise in the context.

In the disclosure, an expression "have," "may have," "include," "may include" or the like, indicates existence of a corresponding feature (for example, a numerical value, a function, an operation or a component such as a part), and does not exclude existence of an additional feature.

In the disclosure, expressions "A or B," "least one of A and/or B," "one or more of A and/or B" and the like, may include all possible combinations of items enumerated together. For example, "A or B," "at least one of A and B" or "at least one of A or B" may indicate all of 1) a case where at least one A is included, 2) a case where at least one B is included, or 3) a case where both of at least one A and at least one B are included.

Expressions "first," "second" and the like, used in the disclosure may indicate various components regardless of a sequence or importance of the components. These expressions are used only to distinguish one component from another component, and do not limit the corresponding components.

In case that any component (for example, a first component) is mentioned to be "(operatively or communicatively) coupled with/to" or "connected to" another component (for example, a second component), it is to be understood that any component is directly coupled to another component or may be coupled to another component through still another component (for example, a third component).

On the other hand, in case that any component (for example, a first component) is mentioned as being "directly coupled" or "directly connected" to another component (for example, a second component), it is to be understood that still another component (for example, a third component) does not exist between any component and another component.

An expression "configured (or set) to" used in the disclosure may be replaced by an expression "suitable for," "having the capacity to," "designed to," "adapted to," "made to" or "capable of" based on a situation. The expression "configured (or set) to" may not necessarily indicate "specifically designed to" in hardware.

Instead, an expression a "device configured to" in any situation may indicate that the device may "perform~" together with another device or component. For example, "a processor configured (or set) to perform A, B and C" may indicate a dedicated processor (for example, an embedded processor) for performing the corresponding operations or a generic-purpose processor (for example, a central processing unit (CPU) or an application processor) that may perform the corresponding operations by executing one or more software programs stored in a memory device.

In the embodiments, a "module" or a "~er/or" may perform at least one function or operation, and be implemented by hardware or software, or be implemented by a combination of hardware and software. In addition, a plurality of "modules" or a plurality of "~ers/ors" may be integrated in at least one module and implemented by at least one processor except for a "module" or an "~er/or" that needs to be implemented by specific hardware.

Meanwhile, various elements and regions in the drawings are schematically shown.

Hereinafter, the embodiments of the disclosure are described in detail with reference to the accompanying drawings for those skilled in the art to which the disclosure pertains to easily practice the disclosure.

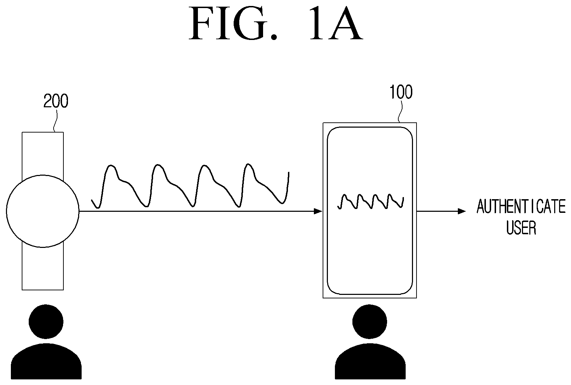

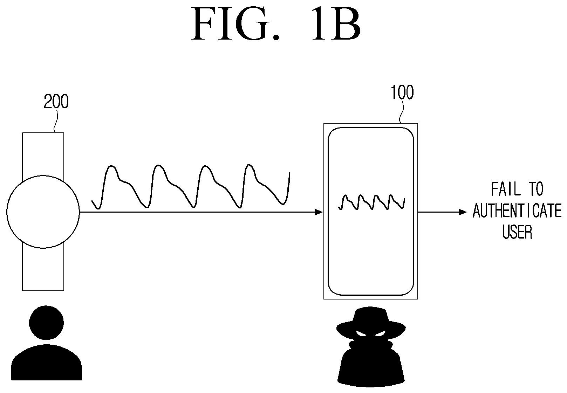

FIGS. 1A and 1B are views provided to describe a user authentication system according to an embodiment.

When a user touch for a first pulse wave sensor included in an electronic apparatus 100 is detected, the electronic apparatus 100 may obtain a first pulse wave signal through the first pulse wave sensor, and a wearable device 200 may obtain a second pulse wave signal through a second pulse wave sensor.

In this case, the second pulse wave signal may be a pulse wave signal obtained based on time stamp information corresponding to the first pulse wave signal.

The electronic apparatus 100 may authenticate the user by comparing the first pulse wave signal and the second pulse wave signal.

For example, as illustrated in FIG. 1A, when the electronic apparatus 100 identifies that the first pulse wave signal and the second pulse wave signal are obtained from the same user by comparing the first pulse wave signal and the second pulse wave signal, the electronic apparatus 100 may authenticate the user.

However, as illustrated in FIG. 1B, when the electronic apparatus 100 identifies that the first pulse wave signal and the second pulse wave signal are obtained from different users by comparing the first pulse wave signal and the second pulse wave signal, the user authentication may fail.

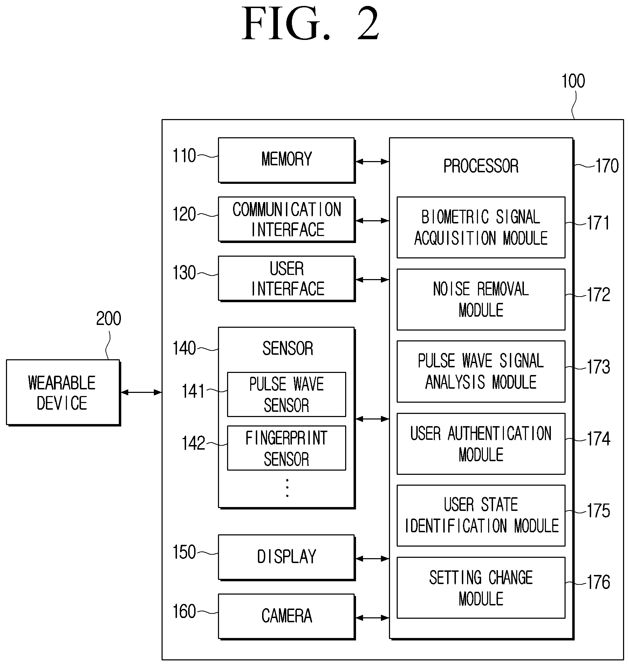

FIG. 2 is a block diagram provided to describe the configuration of the electronic apparatus 100 according to an embodiment.

The electronic apparatus 100 may include a memory 110, a communication interface 120, a user interface 130, a sensor 140, a display 150, and a processor 170. The electronic apparatus 100 may omit some of the above components, or may further include other components. The electronic apparatus 100 may be a smartphone, but this is only an example. The electronic apparatus 100 may be implemented through various devices.

The memory 110 may store at least one instruction regarding the electronic apparatus 100. The memory 110 may store an Operating System (O/S) for driving the electronic apparatus 100. In addition, the memory 110 may store various software programs or applications for the electronic apparatus 100 to operate according to various embodiments. In addition, the memory 110 may store a semiconductor memory such as a flash memory or a magnetic storage medium such as a hard disk.

Specifically, the memory 110 may store various software modules for the electronic apparatus 100 to operate according to various embodiments, and the processor 170 may control the operation of the electronic apparatus 100 by executing the various software modules stored in the memory 110. In other words, the memory 110 may be accessed by the processor 170, and reading/recording/modifying/deleting/updating data by the processor 170 may be performed.

In addition, the memory 110 may store fingerprint information of the user for authenticating the user. Further, the memory 110 may store at least one algorithm for identifying the user's state (e.g.: whether the user is in a sleeping state or whether the user is conscious) through the user's face image obtained through the camera 110.

Meanwhile, the term 'memory 110' herein may be meant to include the memory 110, a ROM (not shown) or a RAM (not shown) in the processor 170, or a memory card (not shown) (e.g.: a micro SD card or a memory stick) mounted in the electronic apparatus 100.

The communication interface 120 may include circuitry and communicate with an external apparatus and a server. The communication interface 120 may perform communication with the external apparatus or the server based on a wired or wireless communication method. The communication interface 120 may include a wireless-fidelity (Wi-Fi) module (not shown), a Bluetooth module (not shown), an infrared (IR) module, a local area network (LAN) module, an Ethernet module, or the like. Here, each communication module may be implemented in the form of at least one hardware chip. In addition to the above-described communication methods, a wireless communication module may include at least one communication chip performing communication based on various wireless communication standards such as zigbee, universal serial bus (USB), mobile industry processor interface camera serial interface (MIPI CSI), third generation (3G), 3rd generation partnership project (3GPP), long term evolution (LTE), LTE advanced (LTE-A), 4th generation (4G), and 5th generation (5G). However, this configuration is only an example, and the communication interface 120 may use at least one communication module among various communication modules.

The user interface 130 is configured to receive a user command for controlling the electronic apparatus 100. The user interface 130 may be implemented as a device such as a button, a touch pad, a mouse, or a keyboard, or may be implemented as a touch screen that may also perform a display function and a manipulation input function. Here, the button may be provided in various types such as a mechanical button, a touch pad, a wheel, and the like, which are formed in arbitrary areas such as a front portion, a side portion, or a back portion of an exterior of a main body of the electronic apparatus 100. The electronic apparatus 100 may obtain various user inputs through the user interface 130. The electronic apparatus 100 may identify that the user is in a sleeping state through a user input that is input through the user interface 130.

The sensor 140 may include a pulse wave sensor 141 and a fingerprint sensor 142, but is not limited thereto. The sensor 140 may include a sensor for obtaining the user's various biometric signals. For example, the sensor 140 may include a motion sensor (not shown), and the motion sensor may detect the user's movement.

The pulse wave sensor 141 is a sensor for sensing the user's pulse wave signal, and may be a Photoplethysmography (PPG) sensor. Here, the pulse wave signal may be a Photoplethysmography (PPG) signal.

A pulse wave is a pulsating waveform that appears as blood waves in the heart, and there may be changes in blood flow and subsequent changes in blood vessel volume due to the relaxation and contraction of the heart. Each time the heart beats, blood vessels expand due to pressure pulses and thus, the arterial blood flow varies, resulting in changes in light transmittance or reflectance. Here, based on the changes in light transmittance and reflectance, the pulse wave sensor 141 may sense a pulse wave signal.

Specifically, the pulse wave sensor 141 may obtain a pulse wave signal by sensing an optical signal in time series through an optical sensor that receives the light irradiated from a light source and then scattered or reflected from the user's blood vessels and herein, the optical sensor may be implemented as a photo diode, a photo transistor, an image sensor, or the like.

The electronic apparatus 100 may authenticate the user by comparing the user's first pulse wave signal obtained through the pulse wave sensor 141 and the second pulse wave signal detected through sensing from the pulse wave sensor included in the wearable device 200.

The fingerprint sensor 142 may obtain the user's fingerprint information. The fingerprint information obtained through the fingerprint sensor 142 is stored as image information, and after being compared with the user's fingerprint information pre-stored in the memory 110, may be used to authenticate the user of the electronic apparatus 100 according to a match score (e.g.: matching rate or similarity). The fingerprint information obtained through the fingerprint sensor 420 may be compressed and stored, and may be stored in the memory 110 as the user's authentication information for future user authentication. The fingerprint information extracted through the fingerprint sensor 420 may be stored in the memory 110 as a feature template.

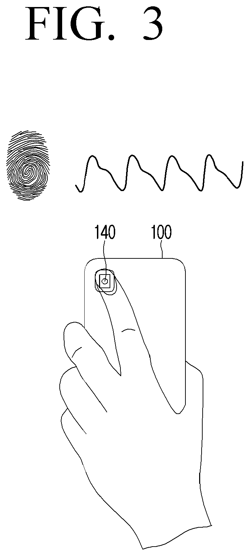

The pulse wave sensor 141 or the fingerprint sensor 142 may be placed on the back of the electronic apparatus 100 as illustrated in FIG. 3, but is not limited thereto. The pulse wave sensor 141 or the fingerprint sensor 142 may be placed to cover at least some areas or the entire areas of the display 150. The pulse wave sensor 141 or the fingerprint sensor 142 may obtain the user's pulse wave signal or fingerprint information at the same time when the user applies a touch input to the pulse wave sensor 141 or the fingerprint sensor 142.

Hereinafter, the description will be focused on the embodiments of sensing the pulse wave signal or fingerprint information of a user (or a subject being measured) and authenticating the user, but the technical idea of the present disclosure is not limited thereto. The technical idea of the present disclosure may be applied when sensing various biometric signals (e.g.: an Electrocardiography (ECG) signal, an Electromyography (EMG) signal) or receiving them from the wearable device 200 and using them to authenticate a user.

The display 150 may be implemented as a display including a self-light emitting element or a display including a non-light emitting element and a backlight. For example, the display may be implemented in various types of displays such as a liquid crystal display (LCD), an organic light emitting diodes (OLED) display, a light emitting diodes (LED), a micro light emitting diode (micro LED), a mini LED, a plasma display panel (PDP), a quantum dot (QD) display, a quantum dot light-emitting diode (QLED). The display 150 may also include a driving circuit, a backlight unit, and the like, which may be implemented in a form such as an a-si thin film transistor (TFT), a low temperature poly silicon (LTPS) TFT, an organic TFT (OTFT), or the like. In particular, the display 150 may display an image obtained through the camera 160.

The camera 160 is configured to photograph a subject to generate a captured image. Here, the captured image may include both a moving image and a still image. The camera 160 may obtain an image of the exterior of the electronic apparatus 100, and may be implemented as a lens, an infrared sensor, etc.

In particular, the camera 160 may be arranged to captures images of the front or rear direction of the electronic apparatus 100, and may generate an image by capturing a user present at the front or rear of the electronic apparatus 100. In particular, the camera 160 may scan the user's face.

The processor 170 may control the overall operations and functions of the electronic apparatus 100. Specifically, the processor 170 is connected to the configuration of the electronic apparatus 100 including the memory 110, and as described above, by executing at least one instruction stored in the memory 110, may control the overall operations of the electronic apparatus 100.

The processor 170 may be implemented in various ways. For example, the processor 170 may be implemented as at least one of an Application Specific Integrated Circuit (ASIC), an embedded processor, a microprocessor, hardware control logic, a haredware Finite State Machine (FSM), or a Digital Signal Processor (DSP). Meanwhile, the term 'processor 170' in the present disclosure may be meant to include a Central Processing Unit (CPU), a Graphic Processing Unit (GPU), a Main Processing Unit (MPU), etc.

The processor 170 may include a biometric signal acquisition module 171, a noise removal module 172, a biometric signal analysis module 173, a user authentication module 174, a user state identification module 175, and a setting change module 176. A plurality of modules according to an embodiment may be implemented as software modules or hardware modules, and when the plurality of modules are implemented as software modules, the processor 170 may access the software modules by loading the software modules stored in the memory 110.

When the user's touch for the pulse wave sensor 141 included in the electronic apparatus 100 is detected, the biometric signal acquisition module 171 may obtain a first pulse wave signal through the pulse wave sensor 141 and receive a second pulse wave signal detected through a pulse wave sensor included in the wearable device 200 from the wearable device 200.

The biometric signal acquisition module 171 may transmit time stamp information corresponding to the first pulse wave signal, measurement time, and a signal for requesting the second pulse wave signal to the wearable device 200. Here, the time stamp information may be time stamp information regarding the time when the user's touch is detected through the pulse wave sensor 141, the time when the user's touch is detected, the time after a predetermined time from the time when the user's touch is detected, or the time when the first pulse wave signal begins to be obtained through the pulse wave sensor 141.

The measurement time may be a value that is pre-stored in the memory 110, but is not limited thereto. The measurement time may be a value input through the user interface 130, the time when the user's touch is detected through the pulse wave sensor 141 or the time when a pulse wave signal is measured through the pulse wave sensor 141.

The second pulse wave signal may be obtained based on the time stamp information and the measurement time.

For example, when the time stamp information corresponding to the first pulse wave signal is 'Mon 11-08-2021 10:00' and the measurement time is '10 seconds', the second pulse wave signal may be a pulse wave signal measured for 10 seconds from 10 O'clock, Monday, November 8, 2021.

The wearable device 200 may obtain the second pulse wave signal and transmit it to the electronic apparatus 100 based on the received time stamp information and measurement time, but is not limited thereto. The wearable device 200 may transmit the second pulse wave signal corresponding to the time stamp information and measurement time received from the electronic apparatus 100 from among pre-obtained pulse wave signals, to the electronic apparatus 100.

When the user's touch for the fingerprint sensor 142 included in the electronic apparatus 100 is detected, the biometric signal acquisition module 171 may obtain the user's fingerprint information through the fingerprint sensor 142.

The noise removal module 172 may remove noise included in a pulse wave signal using a filter. Specifically, the noise removal module 172 may remove noise of the first pulse wave signal and the second pulse wave signal using a band pass filter. For example, when the pulse wave signal is a signal sampled at a sampling rate of 10Hz, the frequency band of the pulse wave signal may be 0 to 5Hz. In this case, assuming that a person's heart rate is 60BPM(1Hz) to 150BPM(2.5Hz), frequency bands other than 1Hz to 2.5Hz may be noise frequency bands. Based on a band pass filter that passes signals with a frequency of 1Hz to 2.5Hz, the noise removal module 172 may obtain the first pulse wave signal and the second pulse wave signal.

The biometric signal analysis module 173 may compare the first pulse wave signal and the second pulse wave signal to identify whether the user of the electronic apparatus 100 (i.e., the user corresponding to the first pulse wave signal) and the user of the wearable device 200 (i.e., the user corresponding to the second pulse wave signal) are identified as the same user. When the user of the electronic apparatus 100 and the user of the wearable device 200 are identified as the same user, the user authentication module 174 may authenticate the user.

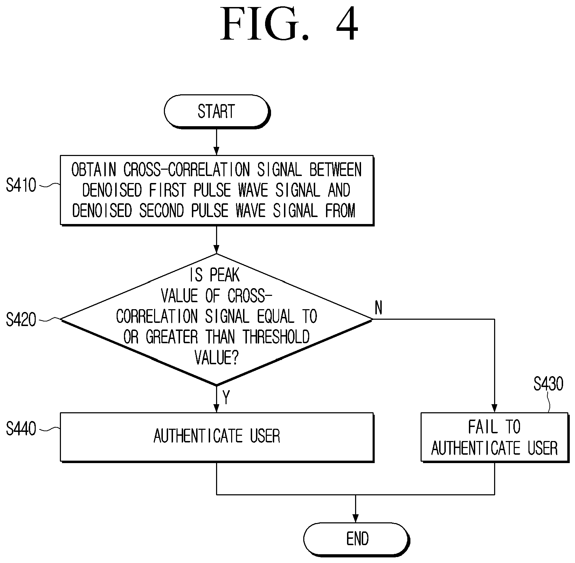

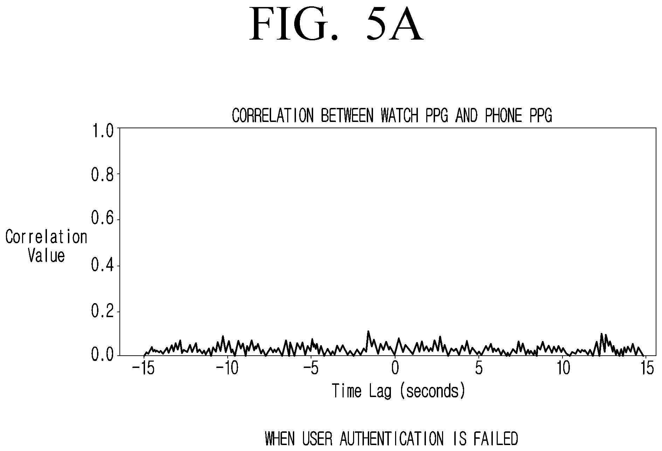

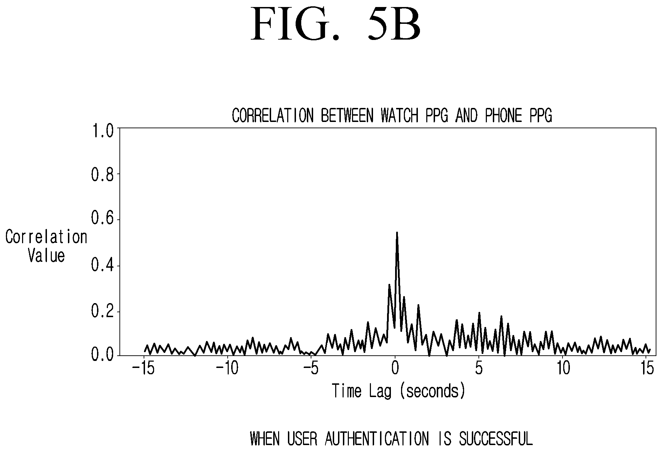

Specifically, referring to FIG. 4, the biometric signal analysis module 173 may obtain a cross correlation signal indicating correlation between the first pulse wave signal and the second pulse wave signal (S410). Here, the obtained cross correlation signal may be as shown in FIGS. 5A and 5B. Meanwhile, hereinafter, the description will focus on the embodiments of removing noise of the first pulse wave signal and the second pulse wave signal, comparing the first pulse wave signal and the second pulse wave signal and authenticating the user, but the technical idea of the present disclosure is not limited thereto. The technical idea of the present disclosure may be applied when authenticating the user by comparing the first pulse wave signal and the second pulse wave signal without removing noise or skipping the step of removing noise in one of the first pulse wave signal and the second pulse wave signal.

The cross correlation signal is a signal for obtaining a correlation (or degree of correlation, correlation, similarity) between two signals. Here, the cross correlation signal may be obtained by a cross correlation function as shown in Mathematical Equation 1 below.

Mathematical Equation 1

$$S3(\tau) = \int_{-\infty}^{\infty} S1(t)S2(t+\tau)d\tau$$

In Mathematical Equation 1, S1 may represent the first pulse wave signal, S2 may represent the second pulse wave signal, and S3 may represent a cross correlation signal.

The biometric signal analysis module 173 may obtain the correlation between the first pulse wave signal and the second pulse wave signal through the signal characteristics of the obtained cross correlation signal. Here, the correlation between the first pulse wave signal and the second pulse wave signal may correspond to at least one peak value of the cross correlation signal. Specifically, the biometric signal analysis module 173 may identify at least one peak value from the obtained cross correlation signal. For example, the biometric signal analysis module 173 may identify the peak value with the largest absolute value from among peak values in the cross correlation signal. Alternatively, the biometric signal analysis module 173 may identify the peak value at the point where the time lag is 0 or the peak value at the point closest to the point where the time lag is 0 in the cross correlation signal. Here, the higher the degree of correlation between the first pulse wave signal and the second pulse wave signal (i.e., the higher the similarity), the greater the absolute value of the peak value identified in the cross correlation signal.

The user authentication module 174 may authenticate the user based on the cross correlation signal (or signal characteristics of the cross correlation signal). Specifically, the user authentication module 174 may identify whether the peak value of the cross correlation signal is equal to or greater than a first threshold value (S420). Here, when the peak value of the cross correlation signal is less than the first threshold value (S420-N), the user authentication module 174 may identify that user authentication has failed (S430). When the peak value of the cross correlation signal is equal to or greater than the first threshold value (S420-Y), the user authentication module 174 may authenticate the user (S440).

For example, when the threshold value is 0.5, user authentication may fail when the cross correlation signal is obtained as illustrated in FIG. 5A, and user authentication may succeed when the cross correlation signal is obtained as illustrated in FIG. 5B.

Meanwhile, according to the above description, the user authentication module 174 authenticates the user based on the peak value of the cross correlation signal obtained by the biometric signal analysis module 173, but this is only an example. According to another embodiment, the user authentication module 174 may authenticate the user based on the deviation score between the first pulse wave signal and the second pulse wave signal, which is obtained by the biometric signal analysis module 173.

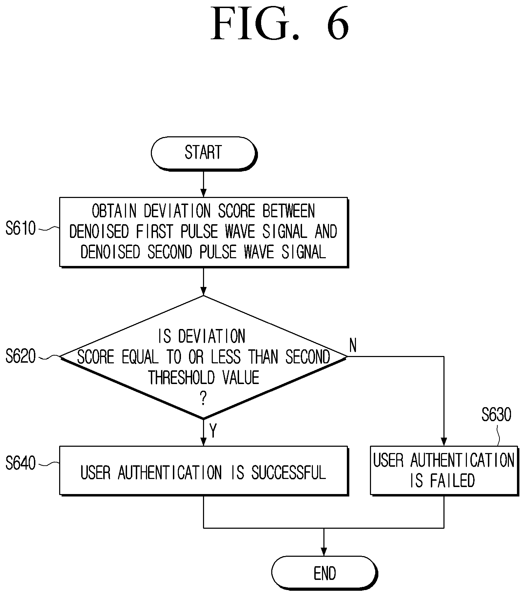

Specifically, referring to FIG. 6, the biometric signal analysis module 173 may obtain the deviation score between the first pulse wave signal and the second pulse wave signal by comparing a peak-to-peak interval (PPI) between the first pulse the first pulse wave signal and the second pulse wave signal, a pulse interval (PI) or a systolic peak (SP) (S610).

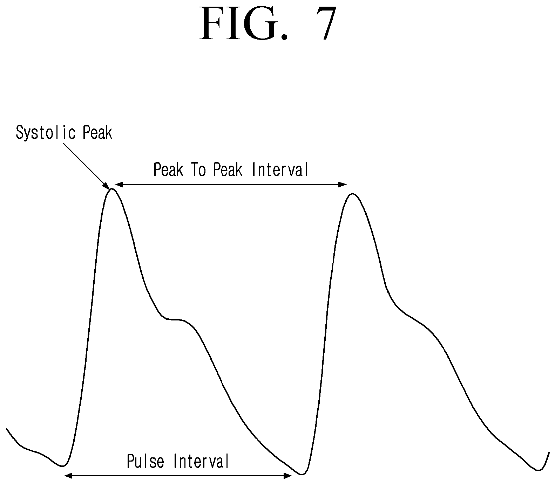

Here, the peak-to-peak interval (PPI), the pulse interval (PI) or the systolic peak (SP) can be obtained as illustrated in FIG. 7, and the systolic peak may mean the point where the pulse wave value is the highest.

The biometric signal analysis module 173 may obtain the deviation score by Mathematical Equation 2 below.

Mathematical Equation 2

$$Dev(PPI)= |\ ((PPI,100)\text{-}(PPI,200))/(PPI,100)\ |$$

$$Dev(PPI)= |\ ((PI,100)\text{-}(PI,200))/(PI,100)\ |$$

$$Dev(PPI)= |\ ((SP,100)\text{-}(SP,200))/(SP,100)\ |$$

Dev(PPI), Dev(PI) and Dev(SP) may mean the deviation score according to the interval between the first pulse wave signal and the second pulse wave signal, the deviation score according to the pulse period and the deviation score according to the systole, respectively. (PPI,100), (PPI,200), (PI,100), (PI,200), (SP,100), and (SP,200) may mean the peak-to-peak interval of the electronic apparatus 100, the peak-to-peak interval of the wearable device 200, the pulse period of the electronic apparatus 100, the pulse period of the wearable device 200, the systolic peak of the electronic apparatus 100, and the systolic peak of the wearable device 200, respectively.

Here, the deviation score may mean one of Dev(PPI), Dev(PI) and Dev(SP), but is not limited thereto. The deviation score may be obtained in various ways such as the weighted average value of Dev(PPI), Dev(PI) and Dev(SP) using Mathematical Equation 3 below.

Mathematical Equation 3

$$\text{Deviation Score} = a\text{*}Dev(PPI)+b\text{*}Dev(PI)+c\text{*}Dev(SP)$$

Here, a, b and c may be arbitrary real numbers.

The user authentication module 174 may identify whether the deviation score is equal to or less than a second threshold value (S620).

When the deviation score exceeds the second threshold value, the user authentication module 174 may identify that user authentication has failed (S630).

When the deviation score is equal to or less than the second threshold value, the user authentication module 174 may authenticate the user (S640).

Meanwhile, the user authentication module 174 may identify the user based on the first pulse wave signal and the second pulse wave signal. Specifically, the memory 110 may store identification information (or identity information) of a registered user and information regarding a third pulse wave signal, and the user authentication module 174 may identify whether the user of the electronic apparatus 100 or the wearable device 200 is a registered user by comparing the first pulse wave signal (or the first pulse wave signal) or the second pulse wave signal (or the second pulse wave signal) and the third pulse wave signal. When it is identified that the user is a registered user, the user authentication module 174 may obtain the user's identification information (identity information).

Here, the method of comparing the first pulse wave signal or the second pulse wave signal and the third pulse wave signal may be the same as the method of comparing the first pulse wave signal and the second pulse wave signal. Meanwhile, as described above, the user authentication module 174 may identify the user based on a pulse wave signal, but is not limited thereto. Specifically, the memory 110 may store identification information of a registered user and various biometric signal information(e.g., fingerprint information), and the user authentication module 174 may identify the user of the electronic apparatus 100 or the wearable device 200 by comparing biometric signal information obtained through the electronic apparatus 100 or the wearable device 200 and the biometric signal information stored in the memory 100 and identify whether the identified user is a registered user.

Even if the user of the electronic apparatus 100 and the user of the wearable device 200 are identified as the same user, when the identified user is identified as not a registered user, user authentication may fail. When the identified user is identified as a registered user, the user authentication module 174 may authenticate the user. Accordingly, even if the user of the electronic apparatus 100 and the user of the wearable device 200 are identified as the same user by comparing the first pulse wave signal and the second pulse wave signal, by additionally identifying whether the user is the same user of the electronic apparatus 100, the security of the user authentication step can be further strengthened.

Meanwhile, it has been described above that the user authentication module 174 authenticates the user based on the user's pulse wave signal obtained through the pulse wave sensor 141, but the present disclosure is not limited thereto. The user authentication module 174 may authenticate the user by additionally using the user's fingerprint information obtained through the fingerprint sensor 142.

Specifically, the user authentication module 174 may obtain the user's fingerprint information through the fingerprint sensor 142. When the user's fingerprint information obtained through the fingerprint sensor 142 and the fingerprint information stored in the memory 110 are the same and the peak value is equal to or greater than the first threshold value or the deviation score is equal to or less than the second threshold value, the user authentication module 174 may authenticate the user. Through the above-described method, the user authentication module 174 may authenticate the user in a method with more enhanced security.

When the user is authenticated, the setting change module 176 may change the setting state of the electronic apparatus 100.

For example, when the user is authenticated while the electronic apparatus 100 is in a locked state, the setting change module 176 may release the locked state of the electronic apparatus 100.

Alternatively, when the user is authenticated while the electronic apparatus 100 is in a child lock state, the setting change module 176 may release the child lock state of the electronic apparatus 100.

Alternatively, when the user is authenticated while a specific folder stored in the memory 110 is in a locked state, the setting change module 176 may allow the user to release the locked state of the specific folder and access the specific folder.

In addition, the setting change module 176 may change the setting state of the electronic apparatus 100 based on the user's identification information. In other words, the setting change module 176 may change the setting state differently depending on the identification information of the authenticated user.

For example, when a first user is authenticated while the electronic apparatus 100 is in a locked state and a child lock state, the setting change module 176 may release the locked state and the child lock state, but when a second user is authenticated while the electronic apparatus 100 is in a locked state and a child lock state, the setting change module 176 may release only the locked state and maintain the child lock state.

Meanwhile, the setting change module 176 may change the setting state of the electronic apparatus 100 based on the user's state.

The user state identification module 175 may identify the user's state. Here, the user may be the user of the electronic apparatus 100 or the user of the wearable device 200. The user's state may mean whether the user is in a sleeping state or whether the user is conscious.

When the user state identification module 175 identifies that the user is in a sleeping state or the user is unconscious, the setting change module 176 may maintain the setting state of the electronic apparatus 100 regardless of whether the user is authenticated.

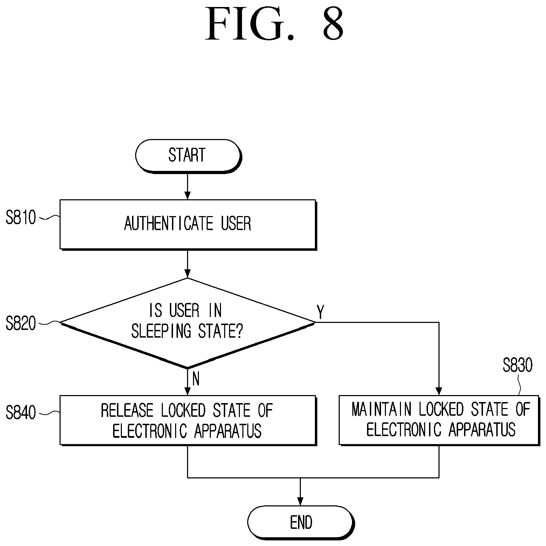

Referring to FIG. 8, the user authentication module 174 may authenticate the user (S810), and the user state identification module 175 may identify whether the user is in a sleeping state (S820). Specifically, it is possible to identify the user's heart rate based on the first pulse wave signal or the second pulse wave signal, and identify whether the user is in a sleeping state based on the identified heart rate. Alternatively, the user state identification module 175 may obtain information regarding whether the user is in a sleeping state through the wearable device 200, or identify that the user is in a sleeping state according to a user input that is input through the user interface 130.

When it is identified that the user is in a sleeping state (S820-Y), the setting change module 176 may maintain the locked state of the electronic apparatus 100 regardless of whether the user is authenticated (S830). When it is identified that the user is not in a sleeping state (S820-N), the setting change module 176 may release the locked state of the electronic apparatus 100.

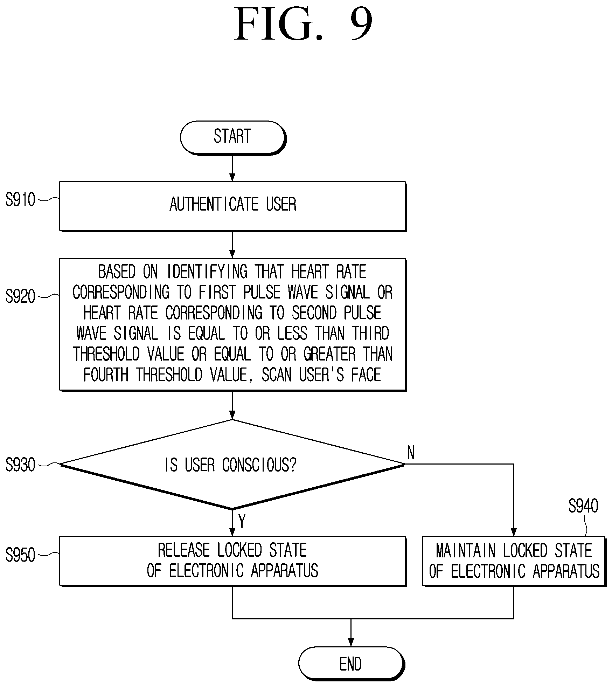

According to another embodiment, as illustrated in FIG. 9, the user authentication module 174 may authenticate the user (S910), and when the heart rate corresponding to the first pulse wave signal or the heart rate corresponding to the second pulse wave signal is equal to or less than a third threshold value or equal to or greater than a fourth threshold value, the user state identification module 175 may scan the user's face through the camera 160 (S920).

The user state identification module 175 may identify whether the user is conscious based on the face scan result (S930). Here, the user state identification module 175 may identify whether the user is conscious based on the user face scan result using an artificial intelligence technology using machine learning such as deep learning and element technologies utilizing machine learning.

When it is identified that the user is unconscious (S930-N), the setting change module 176 may maintain the locked state of the electronic apparatus 100. When it is identified that the user is conscious (S940-Y), the locked state of the electronic apparatus 100 may be released (S950).

Meanwhile, it has been described that whether to release the locked state of the electronic apparatus varies depending on the user's state, but this is only an example. When it is identified that the user is in a sleeping state or unconscious, the setting change module 176 may maintain the setting state of the electronic apparatus 100, such as maintaining the locked state of a specific folder stored in the memory 110 regardless of whether the user is authenticated.

In addition, it has been described that the user is authenticated and the user's state is identified, but this is only an example. The user state identification module 175 may identify the user's state regardless of whether the user is authenticated.

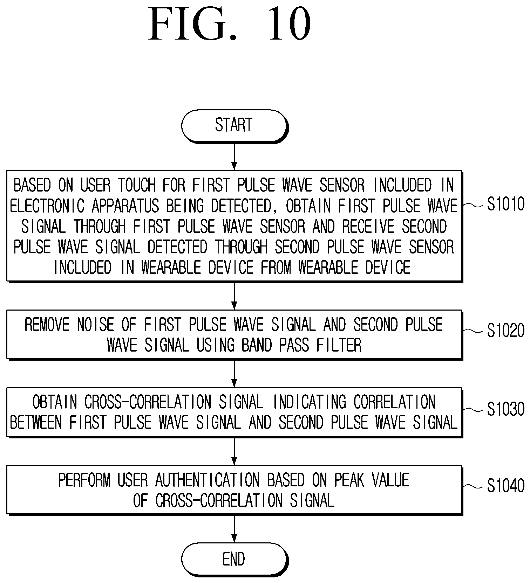

FIG. 10 is a view provided to describe a method of performing user authentication of the electronic apparatus 100 according to an embodiment.

When the user's touch for the first pulse wave sensor included in the electronic apparatus 100 is detected, the electronic apparatus 100 may obtain the first pulse wave signal through the first pulse wave sensor 141 and receive the second pulse wave signal detected through the second pulse wave sensor included in the wearable device 200 from the wearable device 200 (S1010).

The electronic apparatus 100 may remove noise of the first pulse wave signal and the second pulse wave signal using a band pass filter (S1020).

The electronic apparatus 100 may obtain a cross correlation signal indicating a correlation between the first pulse wave signal and the second pulse wave signal (S1030). Alternatively, the electronic apparatus 100 may obtain a deviation score between the first pulse wave signal and the second pulse wave signal.

The electronic apparatus 100 may perform user authentication based on the cross correlation signal (S1040). When the peak value of the cross correlation signal is equal to or less than the first threshold value, the electronic apparatus 100 may authenticate the user. Alternatively, when the deviation score is less than the second threshold value, the electronic apparatus 100 may authenticate the user. When the user is authenticated, the electronic apparatus 100 may change the setting state of the electronic apparatus 100.

Meanwhile, the term "part" or "module" used in the disclosure may include a unit consisting of hardware, software, or firmware, and may be interchangeably used with, for example, terms such as a logic, a logical block, a component, or a circuit. In addition, "a part" or "a module" may be a component constituted as an integrated body or a minimum unit or a part thereof performing one or more functions. For example, a module may be constituted as an application-specific integrated circuit (ASIC).

Also, the various embodiments of the disclosure may be implemented as software including instructions stored in machine-readable storage media, which can be read by machines (e.g.: computers). The machines refer to devices that call instructions stored in a storage medium, and can operate according to the called instructions, and the devices may include an electronic device according to the aforementioned embodiments (e.g.: electronic apparatus 100). In case an instruction is executed by a processor, the processor may perform a function corresponding to the instruction by itself, or by using other components under its control. An instruction may include a code that is generated or executed by a compiler or an interpreter. The machine-readable storage medium may be provided in a form of a non-transitory storage medium. Here, the term "non-transitory" means that the storage medium is tangible without including a signal, and does not distinguish whether data are semi-permanently or temporarily stored in the storage medium.

In addition, according to an embodiment of the present disclosure, the above-described methods according to the diverse embodiments may be included and provided in a computer program product. The computer program product may be traded as a product between a seller and a purchaser. The computer program product may be distributed in a form of a storage medium (for example, a compact disc read only memory (CD-ROM)) that may be read by the machine or online through an application store (for example, PlayStoreTM). In case of the online distribution, at least a portion of the computer program product may be at least temporarily stored in a storage medium such as a memory of a server of a manufacturer, a server of an application store, or a relay server or be temporarily generated.

Each of components (for example, modules or programs) according to various embodiments described above may include a single entity or a plurality of entities, and some of the corresponding sub-components described above may be omitted or other sub-components may be further included in the diverse embodiments. Alternatively or additionally, some components (for example, a module or a program) may be integrated into a single entity to perform the same or similar functions performed by each corresponding components prior to the integration. Operations performed by the modules, the programs, or the other components according to various embodiments may be executed in a sequential manner, a parallel manner, an iterative manner, or a heuristic manner, at least some of the operations may be performed in a different order or be omitted, or other operations may be added.

**Claims**

**1.** A controlling method of an electronic apparatus (100), the method comprising:

based on a user touch for a first pulse wave sensor (141) included in the electronic apparatus being detected, obtaining a first pulse wave signal through the first pulse wave sensor and receiving a second pulse wave signal detected through a second pulse wave sensor included in a wearable device (200) from the wearable device;
removing noise of the first pulse wave signal and the second pulse wave signal using a band pass filter;
obtaining a cross-correlation signal indicating correlation between the denoised first pulse wave signal and the denoised second pulse wave signal; and

authenticating the user based on the cross-correlation signal,

wherein the receiving comprises:
based on the user touch for the first pulse wave sensor (141) included in the electronic apparatus being detected, transmitting time stamp information corresponding to the first pulse wave signal and a signal for requesting the second pulse wave signal to the wearable device; and wherein the second pulse wave signal is obtained based on the time stamp information.

2. The method as claimed in claim 1, wherein authenticating the user comprises, based on a peak value of the cross-correlation signal being equal to or greater than a first threshold value, authenticating the user.

3. The method as claimed in claim 1, further comprising:

obtaining a deviation score between the denoised first pulse wave signal and the denoised second pulse wave signal by comparing a peak-to-peak interval between the denoised first pulse wave signal and the denoised second pulse wave signal, a pulse interval or a systolic peak; and
authenticating the user based on the deviation score.

4. The method as claimed in claim 2, wherein authenticating the user comprises, based on a deviation score being less than a second threshold value, authenticating the user.

5. The method as claimed in claim 1, further comprising:

identifying whether the user is in a sleeping state; and
based on identifying that the user is in a sleeping state, maintaining a locked state of the electronic apparatus regardless of whether the user is authenticated.

6. The method as claimed in claim 1, further comprising:

identifying a heart rate of the user;
based on identifying that the heart rate of the user is equal to or less than a third threshold value or equal to or greater than a fourth threshold value, performing a face scan of the user using a camera of the electronic apparatus;
identifying whether the user is conscious based on the face scan; and
based on identifying that the user is unconscious, maintaining a locked state of the electronic apparatus regardless of whether the user is authenticated.

7. The method as claimed in claim 1, further comprising:
based on the user authentication being failed while the electronic apparatus is in an unlocked state, switching the electronic apparatus to a locked state.

8. The method as claimed in claim 1, further comprising:

obtaining fingerprint information of the user through a fingerprint sensor included in the electronic apparatus, wherein the authenticating the user comprises, based on the fingerprint information matching fingerprint information stored in the electronic apparatus and the peak value being equal to or greater than a first threshold value, authenticating the user.

9. The method as claimed in claim 1, further comprising identifying a user by comparing the first pulse wave signal or the second pulse wave signal with a third pulse wave signal stored in the electronic apparatus.

10. An electronic apparatus (100) comprising:

a first pulse wave sensor (141);
a communication interface (120);
a memory (110) configured to store at least one instruction; and
a processor (170) connected to the memory and configured to control the electronic apparatus,
wherein the processor is configured to, by executing the at least one instruction, cause the electronic apparatus to:

based on a user touch for the first pulse wave sensor being detected, obtain a first pulse wave signal through the first pulse wave sensor and receive a second pulse wave signal detected through a second pulse wave sensor included in a wearable device from the wearable device;

remove noise of the first pulse wave signal and the second pulse wave signal using a band pass filter;

obtain a cross-correlation signal indicating a correlation between the denoised first pulse wave signal and the denoised second pulse wave signal; and

authenticate the user based on the cross-correlation signal,

wherein the processor is configured to cause the apparatus to, based on the user touch for the first pulse wave sensor (141) included in the electronic apparatus being detected, transmit time stamp information corresponding to the first pulse wave signal and a signal for requesting the second pulse wave signal to the wearable device, and

wherein the second pulse wave signal is obtained based on the time stamp information.

11. The apparatus as claimed in claim 10, wherein the processor is configured to cause the apparatus to, based on a peak value of the cross-correlation signal being equal to or greater than a first threshold value, authenticate the user.

12. The apparatus as claimed in claim 10, wherein the processor is configured to cause the apparatus to:

obtain a deviation score between the denoised first pulse wave signal and the denoised second pulse wave signal by comparing a peak-to-peak interval between the denoised first pulse wave signal and the denoised second pulse wave signal, a pulse interval or a systolic peak; and

authenticate the user based on the deviation score.

13. The apparatus as claimed in claim 12, wherein the processor is configured to cause the apparatus to, based on the deviation score being less than a second threshold value, authenticate the user.

## Patentansprüche

1. Steuerungsverfahren einer elektronischen Einrichtung (100), wobei das Verfahren Folgendes umfasst:

basierend darauf, dass eine Benutzerberührung für einen ersten Impulswellensensor (141), der in der elektronischen Einrichtung beinhaltet ist, detektiert wird, Erhalten eines ersten Impulswellensignals durch den ersten Impulswellensensor und Empfangen eines zweiten Impulswellensignals, das durch einen zweiten Impulswellensensor detektiert wird, der in einer tragbaren Vorrichtung (200) beinhaltet ist, von der tragbaren Vorrichtung;

Entfernen von Rauschen des ersten Impulswellensignals und des zweiten Impulswellensignals unter Verwendung eines Bandpassfilters;

Erhalten eines Kreuzkorrelationssignals, das Korrelation zwischen dem entrauschten ersten Impulswellensignal und dem entrauschten zweiten Impulswellensignal angibt; und

Authentifizieren des Benutzers basierend auf dem Kreuzkorrelationssignal,

wobei das Empfangen Folgendes umfasst:

basierend darauf, dass die Benutzerberührung für den ersten Impulswellensensor (141), der in der elektronischen Einrichtung beinhaltet ist, detektiert wird, Übertragen von Zeitstempelinformationen entsprechend dem ersten Impulswellensignal und eines Signals zum Anfordern des zweiten Impulswellensignals an die tragbare Vorrichtung; und

wobei das zweite Impulswellensignal basierend auf den Zeitstempelinformationen erhalten wird.

2. Verfahren nach Anspruch 1, wobei das Authentifizieren des Benutzers basierend darauf, dass ein Spitzenwert des Kreuzkorrelationssignals gleich einem oder größer als ein erster Schwellenwert ist, Authentifizieren des Benutzers umfasst.

3. Verfahren nach Anspruch 1, ferner umfassend:

Erhalten einer Abweichungsbewertung zwischen dem entrauschten ersten Impulswellensignal und dem entrauschten zweiten Impulswellensignal durch Vergleichen eines Spitze-zu-Spitze-Intervalls zwischen dem entrauschten ersten Impulswellensignal und dem entrauschten zweiten Impulswellensignal, eines Impulsintervalls oder einer systolischen Spitze; und
Authentifizieren des Benutzers basierend auf der Abweichungsbewertung.

4. Verfahren nach Anspruch 2, wobei das Authentifizieren des Benutzers basierend darauf, dass eine Abweichungsbewertung weniger als ein zweiter Schwellenwert ist, Authentifizieren des Benutzers umfasst.

5. Verfahren nach Anspruch 1, ferner umfassend:

Identifizieren, ob der Benutzer in einem Schlafzustand ist; und
basierend auf dem Identifizieren, dass der Benutzer in einem Schlafzustand ist, Beibehalten eines verriegelten Zustands der elektronischen Einrichtung unabhängig davon, ob der Benutzer authentifiziert ist.

6. Verfahren nach Anspruch 1, ferner umfassend:

Identifizieren einer Herzfrequenz des Benutzers;
basierend auf dem Identifizieren, dass die Herzfrequenz des Benutzers gleich einem oder weniger als ein dritter Schwellenwert oder gleich einem oder größer als ein vierter Schwellenwert ist, Durchführen einer Gesichtsabtastung des Benutzers unter Verwendung einer Kamera der elektronischen Einrichtung;
Identifizieren, ob der Benutzer bei Bewusstsein ist, basierend auf der Gesichtsabtastung; und
basierend auf dem Identifizieren, dass der Benutzer bewusstlos ist, Beibehalten eines verriegelten Zustands der elektronischen Einrichtung unabhängig davon, ob der Benutzer authentifiziert ist.

7. Verfahren nach Anspruch 1, ferner umfassend:
basierend darauf, dass die Benutzerauthentifizierung fehlgeschlagen ist, während die elektronische Einrichtung in einem entriegelten Zustand ist, Schalten der elektronischen Einrichtung in einen verriegelten Zustand.

8. Verfahren nach Anspruch 1, ferner umfassend:

Erhalten von Fingerabdruckinformationen des Benutzers durch einen Fingerabdrucksensor, der in der elektronischen Einrichtung beinhaltet ist,
wobei das Authentifizieren des Benutzers basierend darauf, dass die Fingerabdruckinformationen mit Fingerabdruckinformationen übereinstimmen, die in der elektronischen Einrichtung gespeichert sind, und der Spitzenwert gleich einem oder größer als ein erster Schwellenwert ist, Authentifizieren des Benutzers umfasst.

9. Verfahren nach Anspruch 1, ferner umfassend Identifizieren eines Benutzers durch Vergleichen des ersten Impulswellensignals oder des zweiten Impulswellensignals mit einem dritten Impulswellensignal, das in der elektronischen Einrichtung gespeichert ist.

10. Elektronische Einrichtung (100), umfassend:

einen ersten Impulswellensensor (141);
eine Kommunikationsschnittstelle (120);
einen Speicher (110), der dazu konfiguriert ist, zumindest eine Anweisung zu speichern; und
einen Prozessor (170), der mit dem Speicher verbunden und dazu konfiguriert ist, die elektronische Einrichtung zu steuern, wobei der Prozessor dazu konfiguriert ist, durch Ausführen der zumindest einen Anweisung die elektronische Einrichtung zu Folgendem zu veranlassen:

basierend darauf, dass eine Benutzerberührung für den ersten Impulswellensensor detektiert wird, Erhalten eines ersten Impulswellensignals durch den ersten Impulswellensensor und Empfangen eines zweiten Impulswellensignals, das durch einen zweiten Impulswellensensor detektiert wird, der in einer tragbaren Vorrichtung beinhaltet ist, von der tragbaren Vorrichtung;
Entfernen von Rauschen des ersten Impulswellensignals und des zweiten Impulswellensignals unter Verwendung eines Bandpassfilters;
Erhalten eines Kreuzkorrelationssignals, das eine Korrelation zwischen dem entrauschten ersten Impulswellensignal und dem entrauschten zweiten Impulswellensignal angibt; und
Authentifizieren des Benutzers basierend auf dem Kreuzkorrelationssignal,
wobei der Prozessor dazu konfiguriert ist, die Einrichtung zu veranlassen, basierend darauf, dass die Benutzerberührung für den ersten Impulswellensensor (141), der in der elektronischen Einrichtung enthalten ist, detektiert wird, Zeitstempelinformationen entsprechend dem ersten Impulswellensignal und ein Signal zum Anfordern des zweiten Impulswellensignals an die tragbare Vorrichtung zu übertragen, und wobei das zweite Impulswellensignal basierend auf den Zeitstempelinformationen erhalten wird.

11. Einrichtung nach Anspruch 10, wobei der Prozessor dazu konfiguriert ist, die Einrichtung zu veranlassen, basierend darauf, dass ein Spitzenwert des Kreuzkorrelationssignals gleich einem oder größer als ein erster Schwellenwert ist, den Benutzer zu authentifizieren.

12. Einrichtung nach Anspruch 10, wobei der Prozessor dazu konfiguriert ist, die Einrichtung zu Folgendem zu veranlassen:

Erhalten einer Abweichungsbewertung zwischen dem entrauschten ersten Impulswellensignal und dem entrauschten zweiten Impulswellensignal durch Vergleichen eines Spitze-zu-Spitze-Intervalls zwischen dem entrauschten ersten Impulswellensignal und dem entrauschten zweiten Impulswellensignal, eines Impulsintervalls oder einer systolischen Spitze; und
Authentifizieren des Benutzers basierend auf der Abweichungsbewertung.

13. Einrichtung nach Anspruch 12, wobei der Prozessor dazu konfiguriert ist, die Einrichtung zu veranlassen, basierend darauf, dass die Abweichungsbewertung weniger als ein zweiter Schwellenwert ist, den Benutzer zu authentifizieren.

## Revendications

1. Procédé de commande d'un appareil électronique (100), le procédé comprenant :

   sur la base de la détection d'un toucher d'utilisateur pour un premier capteur d'onde de pouls (141) compris dans l'appareil électronique, l'obtention d'un premier signal d'onde de pouls par l'intermédiaire du premier capteur d'onde de pouls et la réception d'un deuxième signal d'onde de pouls détecté par l'intermédiaire d'un second capteur d'onde de pouls compris dans un dispositif portable (200) à partir du dispositif portable ;
   l'élimination de bruit du premier signal d'onde de pouls et du deuxième signal d'onde de pouls à l'aide d'un filtre passe-bande ;
   l'obtention d'un signal de corrélation croisée indiquant une corrélation entre le premier signal d'onde de pouls sans bruit et le deuxième signal d'onde de pouls sans bruit ; et
   l'authentification de l'utilisateur sur la base du signal de corrélation croisée,
   dans lequel la réception comprend :

      sur la base de la détection du toucher de l'utilisateur pour le premier capteur d'onde de pouls (141) compris dans l'appareil électronique, la transmission d'informations d'horodatage correspondant au premier signal d'onde de pouls et d'un signal pour demander le deuxième signal d'onde de pouls au dispositif portable ; et
      dans lequel le deuxième signal d'onde de pouls est obtenu sur la base des informations d'horodatage.

2. Procédé selon la revendication 1, dans lequel l'authentification de l'utilisateur comprend, sur la base du fait qu'une valeur de crête du signal de corrélation croisée est supérieure ou égale à une première valeur seuil, l'authentification de l'utilisateur.

3. Procédé selon la revendication 1, comprenant en outre :

   l'obtention d'un score d'écart entre le premier signal d'onde de pouls sans bruit et le deuxième signal d'onde de pouls sans bruit en comparant un intervalle de crête à crête entre le premier signal d'onde de pouls sans bruit et le deuxième signal d'onde de pouls sans bruit, un intervalle de pouls ou une crête systolique ; et
   l'authentification de l'utilisateur sur la base du score d'écart.

4. Procédé selon la revendication 2, dans lequel l'authentification de l'utilisateur comprend, sur la base du fait qu'un score d'écart est inférieur à une deuxième valeur seuil, l'authentification de l'utilisateur.

5. Procédé selon la revendication 1, comprenant en outre :

   le fait d'identifier si l'utilisateur est dans un état de sommeil ; et

sur la base de l'identification du fait que l'utilisateur est dans un état de sommeil, le maintien d'un état verrouillé de l'appareil électronique, que l'utilisateur soit authentifié ou non.

6. Procédé selon la revendication 1, comprenant en outre :

l'identification d'une fréquence cardiaque de l'utilisateur ;
sur la base de l'identification du fait que la fréquence cardiaque de l'utilisateur est inférieure ou égale à une troisième valeur seuil ou supérieure ou égale à une quatrième valeur seuil, la réalisation d'un balayage du visage de l'utilisateur à l'aide d'une caméra de l'appareil électronique ;
le fait d'identifier si l'utilisateur est conscient sur la base du balayage du visage ; et
sur la base de l'identification du fait que l'utilisateur est inconscient, le maintien d'un état verrouillé de l'appareil électronique, que l'utilisateur soit authentifié ou non.

7. Procédé selon la revendication 1, comprenant en outre :
sur la base d'un échec de l'authentification de l'utilisateur alors que l'appareil électronique est dans un état déverrouillé, la commutation de l'appareil électronique à un état verrouillé.

8. Procédé selon la revendication 1, comprenant en outre :

l'obtention d'informations d'empreintes digitales de l'utilisateur par l'intermédiaire d'un capteur d'empreintes digitales compris dans l'appareil électronique,
dans lequel l'authentification de l'utilisateur comprend, sur la base du fait que les informations d'empreintes digitales correspondent à des informations d'empreintes digitales stockées dans l'appareil électronique et que la valeur de crête est supérieure ou égale à une première valeur seuil, l'authentification de l'utilisateur.

9. Procédé selon la revendication 1, comprenant en outre l'identification d'un utilisateur en comparant le premier signal d'onde de pouls ou le deuxième signal d'onde de pouls avec un troisième signal d'onde de pouls stocké dans l'appareil électronique.

10. Appareil électronique (100) comprenant :

un premier capteur d'onde de pouls (141) ;
une interface de communication (120) ;
une mémoire (110) configurée pour stocker au moins une instruction ; et
un processeur (170) connecté à la mémoire et configuré pour commander l'appareil électronique,
dans lequel le processeur est configuré pour, en exécutant l'au moins une instruction, amener l'appareil électronique à :

sur la base de la détection d'un toucher d'utilisateur pour le premier capteur d'onde de pouls, obtenir un premier signal d'onde de pouls par l'intermédiaire du premier capteur d'onde de pouls et recevoir un deuxième signal d'onde de pouls détecté par l'intermédiaire d'un second capteur d'onde de pouls compris dans un dispositif portable à partir du dispositif portable ;
éliminer du bruit du premier signal d'onde de pouls et du deuxième signal d'onde de pouls à l'aide d'un filtre passe-bande ;
obtenir un signal de corrélation croisée indiquant une corrélation entre le premier signal d'onde de pouls sans bruit et le deuxième signal d'onde de pouls sans bruit ; et
authentifier l'utilisateur sur la base du signal de corrélation croisée,
dans lequel le processeur est configuré pour amener l'appareil, sur la base de la détection du toucher de l'utilisateur pour le premier capteur d'onde de pouls (141) compris dans l'appareil électronique, à transmettre des informations d'horodatage correspondant au premier signal d'onde de pouls et un signal pour demander le deuxième signal d'onde de pouls au dispositif portable, et dans lequel le deuxième signal d'onde de pouls est obtenu sur la base des informations d'horodatage.

11. Appareil selon la revendication 10, dans lequel le processeur est configuré pour amener l'appareil, sur la base du fait qu'une valeur de crête du signal de corrélation croisée est supérieure ou égale à une première valeur seuil, à authentifier l'utilisateur.

12. Appareil selon la revendication 10, dans lequel le processeur est configuré pour amener l'appareil à :

obtenir un score d'écart entre le premier signal d'onde de pouls sans bruit et le deuxième signal d'onde de pouls sans bruit en comparant un intervalle de crête à crête entre le premier signal d'onde de pouls sans bruit et le deuxième signal d'onde de pouls sans bruit, un intervalle de pouls ou une crête systolique ; et

authentifier l'utilisateur sur la base du score d'écart.

13. Appareil selon la revendication 12, dans lequel le processeur est configuré pour amener l'appareil, sur la base du fait que le score d'écart est inférieur à une deuxième valeur seuil, à authentifier l'utilisateur.

# FIG. 1A

200

100

AUTHENTICATE
USER

# FIG. 1B

FAIL TO
AUTHENTICATE
USER

# FIG. 2

100

110 — MEMORY

120 — COMMUNICATION INTERFACE

130 — USER INTERFACE

200 — WEARABLE DEVICE

140 — SENSOR

141 — PULSE WAVE SENSOR

142 — FINGERPRINT SENSOR

⋮

150 — DISPLAY

160 — CAMERA

PROCESSOR — 170

BIOMETRIC SIGNAL ACQUISITION MODULE — 171

NOISE REMOVAL MODULE — 172

PULSE WAVE SIGNAL ANALYSIS MODULE — 173

USER AUTHENTICATION MODULE — 174

USER STATE IDENTIFICATION MODULE — 175

SETTING CHANGE MODULE — 176

# FIG. 3

# FIG. 4

START

S410 — OBTAIN CROSS-CORRELATION SIGNAL BETWEEN DENOISED FIRST PULSE WAVE SIGNAL AND DENOISED SECOND PULSE WAVE SIGNAL FROM

S420 — IS PEAK VALUE OF CROSS-CORRELATION SIGNAL EQUAL TO OR GREATER THAN THRESHOLD VALUE?

N

S430 — FAIL TO AUTHENTICATE USER

Y

S440 — AUTHENTICATE USER

END

# FIG. 5A

CORRELATION BETWEEN WATCH PPG AND PHONE PPG

WHEN USER AUTHENTICATION IS FAILED

# FIG. 5B

CORRELATION BETWEEN WATCH PPG AND PHONE PPG

Correlation Value vs Time Lag (seconds)

WHEN USER AUTHENTICATION IS SUCCESSFUL

# FIG. 6

```
                    ┌─────────────┐
                    │    START    │
                    └──────┬──────┘
                           │
                           ▼
         ┌────────────────────────────────────┐
         │   OBTAIN DEVIATION SCORE BETWEEN    │
  S610 ──│ DENOISED FIRST PULSE WAVE SIGNAL AND│
         │  DENOISED SECOND PULSE WAVE SIGNAL  │
         └────────────────┬───────────────────┘
                          │
                          ▼
                     IS DEVIATION                    N
  S620 ──  SCORE EQUAL TO OR LESS THAN SECOND  ──────────┐
                    THRESHOLD VALUE                      │
                         ?                               │
                          │                              │
                          │ Y                      S630  │
                          ▼                              ▼
         ┌────────────────────────────────┐   ┌──────────────────┐
  S640 ──│ USER AUTHENTICATION IS SUCCESSFUL│  │ USER AUTHENTICATION│
         └────────────────┬───────────────┘   │     IS FAILED    │
                          │                    └─────────┬────────┘
                          │                              │
                          └──────────────┬───────────────┘
                                         ▼
                                  ┌─────────────┐
                                  │     END     │
                                  └─────────────┘
```

# FIG. 7

Systolic Peak

Peak To Peak Interval

Pulse Interval

# FIG. 8

```
        ┌─────────┐
        │  START  │
        └────┬────┘
             │
             ▼
S810 ┌──────────────────────┐
     │   AUTHENTICATE USER   │
     └──────────┬───────────┘
                │
                ▼
          ╱────────────╲                    Y
S820 ────<  IS USER IN   >─────────────────────┐
          ╲ SLEEPING STATE?╱                   │
           ╲────────────╱                      │
                │ N                            │  S830
                ▼                              ▼
S840 ┌──────────────────────┐    ┌──────────────────────┐
     │  RELEASE LOCKED STATE │    │ MAINTAIN LOCKED STATE │
     │  OF ELECTRONIC        │    │ OF ELECTRONIC         │
     │  APPARATUS            │    │ APPARATUS             │
     └──────────┬───────────┘    └──────────┬───────────┘
                │                            │
                └──────────┬─────────────────┘
                           ▼
                      ┌─────────┐
                      │   END   │
                      └─────────┘
```

# FIG. 9

START

S910 — AUTHENTICATE USER

S920 — BASED ON IDENTIFYING THAT HEART RATE CORRESPONDING TO FIRST PULSE WAVE SIGNAL OR HEART RATE CORRESPONDING TO SECOND PULSE WAVE SIGNAL IS EQUAL TO OR LESS THAN THIRD THRESHOLD VALUE OR EQUAL TO OR GREATER THAN FOURTH THRESHOLD VALUE, SCAN USER'S FACE

S930 — IS USER CONSCIOUS?   N

S940

Y

S950 — RELEASE LOCKED STATE OF ELECTRONIC APPARATUS

MAINTAIN LOCKED STATE OF ELECTRONIC APPARATUS

END

# FIG. 10

START

BASED ON USER TOUCH FOR FIRST PULSE WAVE SENSOR INCLUDED IN ELECTRONIC APPARATUS BEING DETECTED, OBTAIN FIRST PULSE WAVE SIGNAL THROUGH FIRST PULSE WAVE SENSOR AND RECEIVE SECOND PULSE WAVE SIGNAL DETECTED THROUGH SECOND PULSE WAVE SENSOR INCLUDED IN WEARABLE DEVICE FROM WEARABLE DEVICE ～ S1010

REMOVE NOISE OF FIRST PULSE WAVE SIGNAL AND SECOND PULSE WAVE SIGNAL USING BAND PASS FILTER ～ S1020

OBTAIN CROSS-CORRELATION SIGNAL INDICATING CORRELATION BETWEEN FIRST PULSE WAVE SIGNAL AND SECOND PULSE WAVE SIGNAL ～ S1030

PERFORM USER AUTHENTICATION BASED ON PEAK VALUE OF CROSS-CORRELATION SIGNAL ～ S1040

END

**EP 4 421 663 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20210153757 A1
- US 9558336 B2
- US 9223956 B2